# EUROPEAN PATENT APPLICATION

(11) **EP 3 050 895 A1**
(43) Date of publication of application: **03.08.2016**
(21) Application number: 14850072.1
(22) Date of filing: 26.09.2014
(51) Int. Cl.: C07K 7/06, C07K 1/20, C07K 1/06, C07K 1/04, A61K 38/08, A61P 35/00

(54) **SALT OF POLYPEPTIDE VACCINE, PREPARATION METHOD THEREFOR, AND PHARMACEUTICAL PREPARATION COMPRISING SAID SALT**

(30) Priority: 26.09.2013 CN 201310447114
(71) Applicant: Shenzhen Salubris Pharmaceuticals Co., Ltd., Shenzhen, Guangdong 518000 (CN)
(72) Inventor: TAN, Duanming, Shenzhen Guangdong 518100 (CN); TIAN, Maokui, Shenzhen Guangdong 518100 (CN); SUN, Baojin, Shenzhen Guangdong 518100 (CN)
(74) Representative: Zacco Denmark A/S
(86) International application number: PCT/CN2014/087506
(87) International publication number: WO 2015/043497

(57) **Abstract**

The present invention belongs to the field of pharmaceutical technology and relates to salt of polypeptide vaccine, preparation method therefor, and pharmaceutical preparation comprising said salt, in particular to KIFGSLAFL acetate, its preparation method and pharmaceutical preparations containing such acetate.

## Description

The present invention belongs to the field of pharmaceutical technology and relates to salt of polypeptide vaccine, preparation method therefor, and pharmaceutical preparation comprising said salt, in particular to KIFGSLAFL acetate, the preparation method and a pharmaceutical preparation containing such acetate.

### Background of the invention

Breast cancer (BCa) is the most common cancer diagnosed in female and the second leading factor of death related to cancer for female (RiesLAG et al., SEER Cancer Statistics Review, 1975-2003, National Cancer Institute, Bethesda, MD). During the past 20 years, the major progress for the treatment of breast cancer improves the disease-free survival (DFS) rate significantly. For example, the antibody therapy reactive to tumor-associated antigen has been used for blocking specific cellular process to slow the progression of disease or to prevent the disease recurrence. Although the treatment of breast cancer progressed in recent years, a fair amount of patients ultimately died from recurrent diseases. Therefore, the treatment for preventing, slowing or inhibiting recurrent disease is necessary.

Vaccine is the noticeable model for such treatment and prevention because of the convenience of administration and the high success rate observed in infectious diseases. The fundamental principle for constructing cancer vaccine is direct theoretically. However, the success rate is limited in the development of effective cancer vaccine for solid tumor practically.

KIFGSLAFL (nonapeptide with the amino acid sequence of Lys-Ile-Phe-Gly-Ser-Leu -Ala-Phe-Leu, also known as HER2/neu 369-377), the peptide sequence of HER2/neu proto-oncogene family, responds to cytotoxicity T lymphocyte (CTL), is intended for vaccine aiming at preventing and/or treating cancer and is relatively used in clinical study at present (Zaks T et al., Immunization with a peptide epitope (369-377) from HER-2/neu leads to peptide specific cytotoxic T lymphocytes that fail to recognize HER-2/neu+tumors, Cancer Research, 1998, 58(21):4902-8; Knutson KL et al., Immunization of cancer patients with HER-2/neu, HLA-A2 peptide, p369-377, fesults in short-lived peptide-specific immunity, Clin Cancer Res 2002, 8(5): 1014-1018).

However, KIFGSLAFL samples used in avaliable clinical trial of disclosured technologies are in the state of DMSO solution, with poor stability at room temperature and should be kept at low temperature of -20°C and thawed before using. So far, no compound or mixture of such polypeptide stable at room temperature has been reported.

### Detailed description of the invention

One aim of the invention is to provide KIFGSLAFL acetate to improve the purity, water solubility and stability of raw materials significantly.

Wherein, the said KIFGSLAFL means nonapeptide with the following amino acid sequence: Lys-Ile-Phe-Gly-Ser-Leu-Ala-Phe-Leu

Which is also known as HER2/neu 369-377, and the molecular formula of the said KIFGSLAFL acetate is C₅₀H₇₈N₁₀O₁₁·C₂H₄O₂.

The above KIFGSLAFL acetate preferably prepared and obtained by the method of the present invention has very high medicinal purity. The purity is ≥95%, preferably ≥98% and more preferably ≥99%. In ensuring the efficacy and safety of medicine, it is important with high commercial development value.

Because large numbers of impurities generate from the synthesis of KIFGSLAFL, there is obvious technical difficulty for preparing high-purity KIFGSLAFL salt and it is difficult to obtain pure single salt with high purity. The invention preferably provides the method for preparing KIFGSLAFL acetate through a lot of experiments, comprising the following steps:

### (1) Preparation of KIFGSLAFL crude

Synthesize KIFGSLAFL peptide resin according to the amino acid sequence of (9→1) with the method of Fmoc solid-phase synthesis, crack it with trifluoroacetic acid to obtain KIFGSLAFL crude, which specifically includes the following steps:
(1.1) Use the acid sensitive resin and connect with Fmoc-Leu-OH (such as Wang resin or 2-chloro-trityl -resin) as raw material to react with piperidine solution, take off Fmoc protecting group and obtain Leu-resin after washing with solvent.
(1.2) In accordance with the solid-phase peptide synthesis method, Leu-resin reacts with Fmoc-protected amino acids successively to obtain the nonapeptide KIFGSLAFL-resin. Conduct transpeptidase reaction with condensing agent successively during the period, wash with solvent, take off Fmoc protecting group, then wash with solvent, carry out the condensation of next Fmoc-protected amino acid.

The Fmoc-protected amino acids for further condensation reaction with Leu-resin are: Fmoc-Phe-OH, Fmoc-Ala-OH, Fmoc-Leu-OH, Fmoc-Ser(tBu)-OH, Fmoc-Gly-OH, Fmoc-Phe-OH, Fmoc-Ile-OH and Fmoc-Lys(Boc)-OH in order.

The condensing agents HATU, HBTU, BOP, PyBOP, DIC/HOBt, DIC/HOAt, etc. are preferable.
(1.3) Treat KIFGSLAFL-resin with trifluoroacetic acid lysis solution for the trifluoroacetic acid solution of KIFGSLAFL, add diethyl ether for precipitation, centrifuge and wash for KIFGSLAFL crude.

The trifluoroacetic acid lysis solution means the carbocation trapping reagent contains trifluoroacetic acid 50-95% (v/v) and the rest dichloromethane or the further added 1-10% (v/v) of water, phenol, p-cresol, anisole, thioanisole, 1,2-dithioglycol, triisopropylchlorosilane (TIS) and other carbocation capture reagents.

### (2) Purification of KIFGSLAFL

Dissolve the KIFGSLAFL crude prepared and obtained from step (1) in the mixed solution of acetonitrile and water, filter, purify the filtrate with reverse-phase high-performance liquid chromatography to obtain pure KIFGSLAFL acetonitrile/water solution.

For the purification of reverse-phase high-performance liquid chromatography, preferably use C4, C8 or C18 alkyl-bonded silica as stationary phase, more preferably C18 alkyl-bonded silica gel; the column temperature is preferably 20-35°C, more preferably 25-30°C; the mobile phase is the acetonitrile/water solution containing trifluoroacetic acid, wherein, the concentration of trifluoroacetic acid is 0.02%-0.5% (v/v), preferably 0.05%-0.2%

(v/v). The preferable volume percentage of acetonitrile in the mobile phase is between 10% and 80% for the gradient elution from low concentration to high concentration, and more preferable between 20% and 50%.

### (3) Salt-transformation (transform into acetate)

Perform reverse-phase high-performance liquid chromatography for the pure KIFGSLAFL prepared and obtained from step (2) with acetic acid in acetonitrile/water solution as the mobile phase to obtain KIFGSLAFL acetate solution.

For the reverse-phase high-performance liquid chromatography used for salt-transformation, preferably use C4 or C8 or C18 alkyl-bonded silica gel as stationary phase, more preferably C18 alkyl-bonded silica gel; the column temperature is preferably 20-35°C, more preferably 25-30°C; the mobile phase is the acetonitrile/water solution containing acetic acid, wherein, the concentration of acetic acid is 0.02%-0.5% (v/v), preferably 0.05%-0.2% (v/v). The preferable volume percentage of acetonitrile in the mobile phase is between 2% and 80% for the gradient elution from low concentration to high concentration, and more preferable to process at least one column volume firstly by isocratic or gradient elution between 2% and 4%, followed by isocratic or gradient elution from low gradient to high gradient between 20% and 50%.

### (4) Solvent removal for solid KIFGSLAFL acetate

One method is to concentrate under reduced pressure and dry the KIFGSLAFL acetate solution prepared and obtained from the above step (3) to obtain the solid KIFGSLAFL acetate.

Another method is to concentrate (under reduced pressure) the KIFGSLAFL acetate solution prepared and obtained from the above step (3), precipitate out KIFGSLAFL acetate, centrifugate for precipitation, vacuum dry to obtain the solid KIFGSLAFL acetate.

Another aim of the invention is that the provided KIFGSLAFL acetate is amorphous solid, and its X-ray diffraction pattern has no crystal characteristic absorption peak. The KIFGSLAFL acetate of amorphous solid can obviously improve water solubility and stability, thus is easier for medicine preparation, in particular for the use of preparation injection.

The invention provides the method for preparing amorphous solid KIFGSLAFL acetate through a lot of experiments, comprising the following steps:
Adjust the pH value of the KIFGSLAFL acetate solution prepared and obtained from the above step (3) to pH 4 with acetic acid, concentrate under reduced pressure until the KIFGSLAFL acetate precipitates out, lower the temperature to below 5°C, centrifugate for precipitation, vacuum dry under 30°C to obtain the uniform KIFGSLAFL acetate amorphous solid.

Wherein, the X-ray diffraction pattern is obtained by the following detection method. Instrument model: Empyrean X-ray diffractometer; test condition: Cu target Kα1 ray, voltage of 40kv, current of 40mA, 2θ range of 3°-50°, divergence slit of 1/32°, anti-scattering slit of 1/16°, anti-scattering slit of 7.5mm, step length of 0.02°, and dwell time per step of 40S.

The said amorphous solid KIFGSLAFL acetate is detected under the IR spectrum detection condition by KBr pellet pressing method, and its infrared absorption reaches peaks at 3288cm⁻¹, 3065cm⁻¹, 2958cm⁻¹, 1632cm⁻¹, 1527 cm⁻¹, 1404 cm⁻¹ and 698cm⁻¹.

The said amorphous solid KIFGSLAFL acetate is detected by ESI mass spectrometry, with the molecular ion peak of 995.9, which is basically the same as the calculated molecular weight for KIFGSLAFL.

The KIFGSLAFL acetate amorphous solid is dissolved in the mixed solution of acetonitrile and water (volume ratio=5:95) and is analyzed under the following detection condition of high-performance liquid chromatography: using C18-bonded silica gel (250×4.6mm, 5µm) as the stationary phase, mobile phase A as acetonitrile, mobile phase B as sodium dihydrogen phosphate solution 0.1mol/L (adjusting the pH value of phosphoric acid to 3.0), conducting gradient elution (towards mobile phase A from 5% to 45% with the elution time of 20min; then changing into 45% isocratic elution of mobile phase A), with the flow velocity of 1mL/min. The detection wavelengths are 195nm (0-8min) and 230nm (8-50min). The elution time for acetic acid and target peptide KIFGSLAFL are approximately 4.6min and 21min, respectively.

Another aim of the invention is to provide a pharmaceutical preparation of peptide vaccine containing the above KIFGSLAFL acetate which is applicable for reformulated into an injectable medical product for patients with pharmaceutically acceptable solvent. The pharmaceutically acceptable solvent comprises water for injection, sterile water, etc.

The efficacy and safety of such pharmaceutical preparation is improved significantly as the KIFGSLAFL acetate of the invention, in particular its amorphous solid has good purity and stability and superior water solubility.

The said pharmaceutical preparation contains one or above medicinal excipients or not. The pharmaceutical preparation of the invention can be in any powder form of parenteral administration, for example, freeze-dried powder, suspension or diary product injection, etc.

The said pharmaceutical preparation can be preferably used for the treatment of breast cancer and breast cancer recurrence.

Unless otherwise specified, the KIFGSLAFL acetate of the invention shall be that with the molecular formula of C₅₀H₇₈N₁₀O₁₁·C₂H₄O₂.

Abbreviations of various reagents in the invention are defined as follows:

| | |
|---|---|
| Fmoc | 9- Fluorenylmethoxycarbonyl |
| DMF | N,N- Dimethylformamide |
| HATU | 2-(7- azabenzotriazole)-N,N,N',N'- tetramethyl-uronium- hexafluophosphate |
| HBTU | benzotriazole -N,N,N',N'- tetramethyl-uronium-hexafluorophosphate |
| BOP | (Benzotriazol-1-yloxy) tris (dimethylamino) phosphonium hexafluophosphate |
| PyBOP | Benzotriazol-1 -yl-oxytripyrrolidino-phosphonium Hexafluorophosphate |
| HOBt | 1-NHydroxybenzotrizole |
| HOAt | N-hydroxy-7-azabenzotriazole |
| DIC | N,N- diisopropylcarbodiimide |
| tBu | tertiary butyl |
| TFA | trifluoroacetic acid |
| TIS | triisopropylchlorosilane |
| v/v | the volume percentage in the same measuring unit, such as mL /mL× 100% |

Compared to the known technologies, the invention has the following prominent advantages and beneficial effects:
1. Compared to KIFGSLAFL and KIFGSLAFL sulfate, the KIFGSLAFL acetate provided in the invention improves the drug purity, stability and water solubility greatly and ensures the medical safety and efficacy sufficiently.
2. The amorphous solid KIFGSLAFL acetate provided in the invention has excellent water solubility and stability, accelerates the redissolving of drug in use by means of injection administration and has better stability for dissolved KIFGSLAFL acetate solution than injection solvent (such as dimethyl sulfoxide). According to the dissolving and stability properties, the amorphous solid KIFGSLAFL acetate provided in the invention belongs to the form more advantageous for preparing pharmaceutical preparation, can sufficiently ensure the convenient use of pharmaceutical preparation, and will be safe and effective for use.
3. The invention provides a method for preparing high-purity KIFGSLAFL acetate and overcomes the technical difficulties in available technologies for preparing salt and improving purity. The method can be carried out in a mild condition, the method condition is easy to be reproduced and can reduce industrialized cost greatly.

### Description of attached figures

Figure 1, an X-ray diffraction spectrum of KIFGSLAFL acetate amorphous solid.
Figure 2, an IR spectrum of KIFGSLAFL acetate amorphous solid.
Figure 3, an ESI mass spectrometry spectra of KIFGSLAFL acetate amorphous solid.

### Embodiments

The following embodiments and attached figures illustrate the invention, but in no way limit the invention.

### Embodiment 1, preparation of KIFGSLAFL crude

1. Weighed 40g of Fmoc-Leu-Wang resin (with the degree of substitution: 0.8 mmol/g), added 300mL of DMF for swelling the resin for 30min. Removed DMF by suction filtration, and added 300mL of 20% piperidine/DMF solution for reaction for 30min. Performed suction filtration, and washed the resin with 6× 150mL of DMF.
2. Added 24.8g of Fmoc-Phe-OH, 8.6g of HOBt and 150mL of DMF, stirred well, added 9.5mL of DIC, and stirred in water bath under 28-30°C for 4∼5h, Detected the resin to be colorless by Kaiser method, performed suction filtration, and washed the resin with 2×200mL of DMF, added 300mL of 20% piperidine/DMF solution for reaction for 30min, performed suction filtration, and washed the resin with 6×200mL of DMF.
3. Referred to step 2, coupled next amino acid in order. The sequence and dosages of subsequent amino acids were: Fmoc-Ala-OH 19.9g, Fmoc-Leu-OH 22.6g, Fmoc-Ser(tBu)-OH 24.5g, Fmoc-Gly-OH 19.0g, Fmoc-Phe-OH 24.8g, Fmoc-Ile-OH 22.6g and Fmoc-Lys(Boc)-OH 30.0g.
4. Washed the resin with dichloromethane and methyl alcohol in turn, vacuum dried to get KIFGSLAFL peptide resin.
5. Weighed 10g of peptide resin, added 100mL of lysis solution (TFA/TIS/H₂O= 95/2.5/2.5, v/v/v), and stirred for 2h at room temperature, filtered the resin, and washed the resin with appropriate quantity of lysis solution, combined the washing liquid with filter liquor, added 1L of diethyl ether under stirring for precipitation, centrifuged and washed with diethyl ether for several time, vacuum dried to get KIFGSLAFL crude.

### Embodiment 2, purification of KIFGSLAFL

Dissolved 5g of KIFGSLAFL crude obtained from embodiment 1 in 100mL of water containing 10% of acetonitrile in volume ratio, and filtered, purified the filtrate by C18 column with the filling height of 250mm and the diameter of 15 mm, conducted gradient elution (B: 20-50%, elution time: 60min) with the mobile phase A of water containing 0.1% of trifluoroacetic acid and the mobile phase B of acetonitrile containing 0.1% of trifluoroacetic acid with the flow velocity of 600mL/min and the detection wavelength of 230nm, collected the main peak ingredients, detected the purification of collected liquid by analytical high-performance liquid chromatography, combined the portions with the purity higher than 98% to get the pure KIFGSLAFL solution, detected the purification of collected liquid by analytical high-performance liquid chromatography, and got the purity of 98.6% and the yield of 45%.

The condition for detecting the contents of trifluoroacetic acid and target peptide by the analytical high-performance liquid chromatography was as follows: high-performance liquid chromatograph: Chuangxin TongHeng LC300, stationary phase: C18-bonded silica gel (250×4.6mm, 5µm), mobile phase A: acetonitrile, and mobile phase B: sodium dihydrogen phosphate solution 0.1mol/L (adjusted the pH value of phosphoric acid to 3.0). Performed gradient elution (transferred mobile phase A from 5% to 45%, with the elution time of 20min; then changed to 45% isocratic elution of mobile phase A) with the flow velocity of 1mL/min and the detection wavelengths of 195nm (0-8min) and 230nm (8-50min). The elution time for trifluoroacetic acid and target peptide were approximately 4.9min and 21min, respectively.

The condition for detecting the purity of target peptide by the analytical high-performance liquid chromatography was as follows: high-performance liquid chromatograph: Chuangxin TongHeng LC300, stationary phase: C18-bonded silica gel (250×4.6mm, 5µm), mobile phase: the mixed liquor of sodium dihydrogen phosphate solution 0.1mol/L (adjusted the pH value of phosphoric acid to 3.0) and acetonitrile (with the volume ratio of 7:3), with the flow velocity of 1mL/min and the detection wavelength of 230nm. The elution time for target peptide was 10min, approximately.

### Embodiment 3, preparation of free KIFGSLAFL peptide

Cooled the KIFGSLAFL solution obtained from embodiment 2 to 0-5°C and dropwise added 5% of NaOH solution until pH value reached to 10 under stirring, concentrated under reduced pressure until precipitated out a mass of gelatinous precipitates, centrifuged, washed with a small amount of ice water, precipitated and vacuum dried to obtain white solid with the purity of 98.1 %.

### Embodiment 4, preparation of KIFGSLAFL acetate solution

The KIFGSLAFL solution obtained from embodiment 2 was purified by C18 column after proper concentration, with the filling height of 250mm and the diameter of 150mm. Eluted with the water solution containing 0.1% of acetic acid in volume ratio and 3% of acetonitrile for 15min, with the flow velocity of 800mL/min. Then carried out gradient elution (B: 20% - 40%, elution time: 50min) with the mobile phase A of water containing 0.1% of acetic acid and the mobile phase B of acetonitrile containing 0.1% acetic acid, with the detection wavelength of 230nm, collected the main peak ingredients for KIFGSLAFL acetate solution with the purity of 99.6% and the yield of 90%.

### Embodiment 5, preparation of KIFGSLAFL acetate amorphous solid

Adjusted pH value of the KIFGSLAFL acetate solution obtained from embodiment 4 to pH 4 by acetic acid, concentrated under reduced pressure until a mass of gelatinous precipitates precipitated out, cooled to 0-5°C, centrifuged for precipitates, vacuum dried under 30°C to obtain white solid.

According to the detection of analytical high-performance liquid chromatography of embodiment 2, the elution time of acetic acid was 4.6min approximately. As being measured, the weight percent of acetic acid in the sample was 5.7%. The purity of KIFGSLAFL was 99.5%.

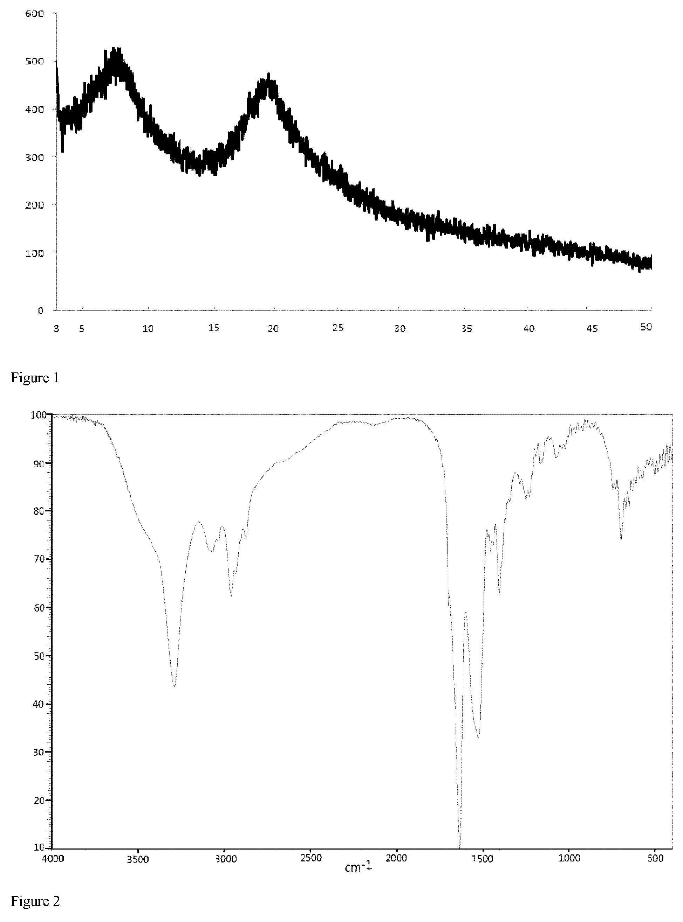

The condition for X-ray diffraction detection was as follows: instrument model: Empyrean X-ray diffractometer; test condition: Cu target Kα1 ray, voltage: 40kv, current: 40mA, 2θ range: 3°-50°, divergence slit: 1/32°, anti-scattering slit: 1/16°, anti-scattering slit: 7.5mm, step length: 0.02°, and the dwell time per step: 40S. X-ray diffraction spectrum was shown in Figure 1.

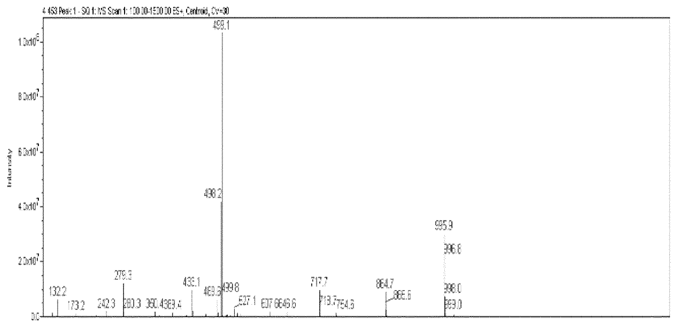

The IR spectrum obtained by KBr pellet pressing method was shown in Figure 2.

The result of ESI mass spectrometric detection was shown in Figure 3, wherein 995.9 m/z peak was M + H⁺, which was basically the same as the calculated molecular weight of KIFGSLAFL.

### Embodiment 6, preparation of KIFGSLAFL·H₂SO₄

Cooled the KIFGSLAFL solution obtained from embodiment 2 to 0-5°C and dropwise added NaOH solution in the weight ratio of 5% until pH value reached to 10 under stirring, concentrated under reduced pressure until precipitated out a mass of gelatinous precipitates, centrifuged and washed with a small amount of ice water, dissolved the solid in the acetonitrile solution with the volume ratio of 50%, added H₂SO₄ in the proportion of 0.5mol H₂SO₄ per mol E75, stirred well (pH=6), and carried out rotary evaporation and concentration under reduced pressure in water bath of 30°C for 2∼3 h, freeze dried to obtain the solid product.

### Embodiment 7, Determination of the stability of KIFGSLAFL and KIFGSLAFL acetate

Studied the stability of the following samples under the sterile condition at 25°C and RH 50%:
Sample 1 was the amorphous solid product of embodiment 5. Sample 2 was the solution of embodiment 4, which was diluted to approximately 1mg/mL with dimethyl sulfoxide and filtered through the filter membrane with the aperture of 0.2 microns. Sample 3 was the solid product of embodiment 3. Sample 4 was the solid product of embodiment 3, which was dissolved in dimethyl sulfoxide, diluted to approximately 1mg/mL and filtered through the filter membrane with the aperture of 0.2 microns. Sample 5 was the solid product of embodiment 6. The purity of each sample was detected by high-performance liquid chromatography, and the results were as follows:

| | Sample 1 | Sample 2 | Sample 3 | Sample 4 | Sample 5 |
|---|---|---|---|---|---|
| Day 0 | 99.5% | 99.6% | 98.1% | 98.1% | 99.2% |
| Day 5 | 99.4% | 98.9% | 97.6% | 96.5% | 98.7% |
| Day 10 | 99.3% | 98.3% | 97.2% | 94.6% | 98.0% |

It could be seen from the experimental results in the table above that: the stabilities of KIFGSLAFL solid (sample 3) and DMSO solution (sample 4) in available technologies were obviously inferior to those of KIFGSLAFL acetate amorphous solid (sample 1) and KIFGSLAFL acetate DMSO solution (sample 2); and the stability of KIFGSLAFL acetate amorphous solid (sample 1) was obviously better than those of DMSO solution (sample 2) and KIFGSLAFL-H₂SO₄ (sample 5).

### Embodiment 8, Determination of solubility

Added the solid products of embodiments 3, 5 and 6 into a certain amount of water for injection of 25°C in small test tubes, respectively, placed in constant temperature shakers for vibration, observed the dissolving conditions. The dissolving conditions were as follows:

| Water | Sample of Embodiment 3 | Sample of Embodiment 5 | Sample of Embodiment 6 |
|---|---|---|---|
| 300 times | > 30min (dissolved incompletely) | < 1min (dissolved completely) | > 30min (dissolved incompletely) |
| 800 times | > 30min (dissolved incompletely) | < 1min (dissolved completely) | > 30min (dissolved incompletely) |

It could be seen from the experimental results in the table above that: the water solubility of KIFGSLAFL acetate amorphous solid (sample of embodiment 5) improved greatly, compared to the KIFGSLAFL solid of available technologies and KIFGSLAFL·H₂SO₄, and was more convenient for the use of preparation.

### Embodiment 9, preparation of peptide vaccine product

Prepared the KIFGSLAFL acetate by the operation of embodiment 5 under sterile condition, weighed accurately (1mg/dose by KIFGSLAFL), subpackaged and sealed in glass bottles to get peptide vaccine product.

### Embodiment 10

Appropriately concentrated the KIFGSLAFL solution obtained from embodiment 2, purified by C18 column, with the filling height of 250mm and the diameter of 150mm. Carried out gradient elution (B: 20% - 40%, elution time: 50min) with mobile phase A of water containing 0.1% of acetic acid in volume ratio and mobile phase B of acetonitrile, with the flow velocity of 800mL/min and the detection wavelength of 230nm, collected the main peak ingredients and obtained KIFGSLAFL solution, concentrated under reduced pressure and freeze dried to obtain white solid. According to the HPLC detection of embodiment 2, the weight percentage of acetic acid was 4.8%, and the weight percentage of trifluoroacetic acid was 1.9%.

### Embodiment 11

Conducted chromatographic purification with the method of embodiment 2 for the KIFGSLAFL crude obtained from embodiment 1 to collect the main peak ingredients. Detected the purity of the collected liquid by analytical high-performance liquid chromatography, combined the portions with purity higher than 92% to get KIFGSLAFL solution, detected the purity of the collected liquid by analytical high-performance liquid chromatography to get the purity of 95.1%, performed salt-transformation by the chromatographic process in embodiment 4 and collected the main peak ingredients to get KIFGSLAFL acetate solution with the purity of 95.3%, concentrated the obtained KIFGSLAFL acetate solution under reduced pressure to dry, vacuum dried under 40°C to obtain white solid. The total yield was 60%. The purity of KIFGSLAFL was detected to be 94.1 % by the HPLC of embodiment 2.

### Embodiment 12

Conducted chromatographic purification with the method of embodiment 2 for the KIFGSLAFL crude obtained from embodiment 1 to collect the main peak ingredients. Detected the purity of the collected liquid by analytical high-performance liquid chromatography, combined the portions with purity higher than 92% to get KIFGSLAFL solution, detected the purity of the collected liquid by analytical high-performance liquid chromatography to get the purity of 95.1%, performed salt-transformation by the chromatographic process in embodiment 4 and collected the main peak ingredients to get KIFGSLAFL acetate solution with the purity of 95.3% and the total yield of 60%, processed with the method in embodiment 5 to obtain white solid, which was amorphous KIFGSLAFL acetate with the purity of 95.3%.

### Embodiment 13

Conducted chromatographic purification with the method of embodiment 2 for the KIFGSLAFL crude obtained from embodiment 1 to collect the main peak ingredients, Detected the purity of the collected liquid by analytical high-performance liquid chromatography, combined the portions with purity higher than 96% to get KIFGSLAFL solution, detected the purity of the collected liquid by analytical high-performance liquid chromatography to get the purity of 97.5%, performed salt-transformation by the chromatographic process in embodiment 4 and collected the main peak ingredients to get KIFGSLAFL acetate solution with the purity of 98.1% and the total yield of 45%, processed with the method in embodiment 5 to obtain white solid, which was amorphous KIFGSLAFL acetate with the purity of 98.0%.

As large numbers of impurities generated from the synthesis of KIFGSLAFL, the industrial preparation of high-purity KIFGSLAFL salt was obviously difficult in technology and it was hard to obtain the pure single salt with high purity. As mentioned in embodiment 10, the synthetic product might easily generate mixed salts, such as acetic acid, trifluoroacetic acid, etc., which were difficult to be separated and purified for the pure single salt.

The inventor could only obtain products with the purity less than 95% by the preferred schemes of conventional separation and purification methods in the field, such as the scheme in embodiment 11 which went against the medical application of product. However, the purity of product was improved to more than 95% or even 98% by preferred purification schemes obtained through a large number of experiments, such as embodiments 12 and 13, which overcame the difficulty of obtaining high-purity KIFGSLAFL salt and made the industrialization and medical application of such product possible.

The above embodiments are preferred execution modes of the invention, but will not be limited by these embodiments. Any other changes, modifications, substitutions, combinations and simplifications without deviation from the spirit and principle of the invention shall be equivalent displacement modes and will be included in the protection scope of the invention.

## Claims

1. KIFGSLAFL acetate, the molecular formula of which is C₅₀H₇₈N₁₀O₁₁·C₂H₄O₂.

2. The acetate according to claim 1, wherein the purity of the KIFGSLAFL acetate is ≥95%, preferably ≥98%, and more preferably ≥99%.

3. The acetate according to claim 1, wherein the KIFGSLAFL acetate is an amorphous solid, the X-ray diffraction pattern of which having no crystal characteristic absorption peak.

4. The acetate according to claim 2, wherein the KIFGSLAFL acetate is an amorphous solid, the X-ray diffraction pattern of which having no crystal characteristic absorption peak.

5. The acetate according to any one of claims 1 to 4, wherein the infrared absorption of the KIFGSLAFL acetate reach peaks at 3288cm⁻¹, 3065cm⁻¹, 2958cm⁻¹, 1632cm⁻¹, 1527 cm⁻¹, 1404 cm⁻¹ and 698cm⁻¹.

6. A pharmaceutical preparation of polypeptide vaccine, wherein containing the KIFGSLAFL acetate specified in any one of claims 1 to 5, and with one or more pharmaceutically acceptable solvent, reformulated into an injectable medical product for patients.

7. The pharmaceutical preparation according to claim 6, wherein the solvent comprises water for injection or sterile water.

8. A method for preparing the KIFGSLAFL acetate according to claim 1 or 2, which comprises the steps as follow:
(1) Synthesize KIFGSLAFL peptide resin according to the amino acid sequence of (9→1) with the method of Fmoc solid-phase synthesis, crack it with trifluoroacetic acid to obtain KIFGSLAFL crude;
(2) Purification of KIFGSLAFL: Dissolve and filter the KIFGSLAFL crude, perform reverse-phase HPLC purification with acetonitrile/water containing trifluoroacetic acid as mobile phase, collect the main peak components;
(3) Salt-transformation: Perform reverse-phase HPLC salt- transformation for the main peak ingredients collected during step (2) with acetic acid in acetonitrile/water as mobile phase, collect the main peak components;
(4) Decompression concentration and drying

9. The method according to claim 8, wherein the purification condition of step (2) and the salt-transformation condition of step (3) respectively use C4, C8 or C18 alkyl-bonded silica gel as stationary phase; the column temperature is 20-35°C; trifluoroacetic acid 0.02%-0.5% (v/v) is used as mobile phase in the purification of step (2), and acetic acid 0.02%-0.5% (v/v) is used as mobile phase in the salt-transformation of step (3).

10. A method for preparing the KIFGSLAFL acetate according to claim 3 or 4, which comprises the steps as follow: adjust the pH value of the KIFGSLAFL acetate solution after salt-transformation to pH 4 with acetic acid, concentrate under reduced pressure until the KIFGSLAFL acetate precipitates out, lower the temperature to below 5°C, centrifuge to obtain precipitation, vacuum dry below 30°C.
